# EUROPÄISCHE PATENTSCHRIFT

(11) **EP 3 456 281 B1**
(45) Veröffentlichungstag und Bekanntmachung des Hinweises auf die Patenterteilung: **05.08.2020**
(21) Anmeldenummer: 18191909.3
(22) Anmeldetag: 31.08.2018
(51) Int. Cl.: A61B 50/30, A61B 90/00, A61B 50/00, A61B 17/00, A61B 17/86

(54) **MEDIZINISCHE VERPACKUNG MIT EINER VERPACKUNGSHÜLSE UND VAKUMIERTER UMVERPACKUNG**
MEDICAL PACKAGE WITH A PACKAGING SLEEVE AND VACUUMED PACKAGING
EMBALLAGE MÉDICAL DOTÉ D'UNE DOUILLE D'EMBALLAGE ET EMBALLAGE EXTÉRIEUR SOUS VIDE

(30) Priorität: 14.09.2017 DE 102017121374
(43) Veröffentlichungstag der Anmeldung: 20.03.2019
(73) Patentinhaber: Rösler IP GmbH, 88138 Hergensweiler (DE)
(72) Erfinder: Rösler, Peter, 88239 Wangen (DE); Rösler, Thiemo, 88239 Wangen (DE)
(74) Vertreter: Riebling, Peter

(56) Entgegenhaltungen:
- EP-A1- 2 108 381
- EP-A1- 3 153 128
- EP-A2- 0 586 209
- DE-A1- 10 301 449
- DE-A1-102007 047 623

## Beschreibung

Gegenstand der Erfindung ist eine medizinische Verpackungshülse für die Aufbewahrung steriler medizinischer Gegenstände mit einer Umverpackung, in deren Innenraum sich ein gegenüber der Atmosphäre verminderter Luftdruck befindet, mit dem Ziel, dass sich die Umverpackung möglichst dicht und faltenfrei um die Verpackungshülse anschmiegt.

Die DE 102009013947A1 zeigt eine Verpackung für Packgutstücke mit einer zu einer Mehrkanthülse geformten Verpackungshülse, die von einer Schrumpffolie umgeben ist. Beim thermischen Schrumpfen der Schrumpffolie können sich jedoch im Übergangsbereich zwischen der Schrumpffolie und der Stirnseite der Mehrkanthülse unerwünschte Falten bilden, bei deren Abknickung die Umverpackung aufbrechen kann.

Die EP1870340A1, die US6547094B1 und die WO200238360A1 zeigen eine Verpackungshülse in der Art eines hohlgeblasenen Behälters, dessen untere Stirnseite abgeflacht ist. Die Abflachung rührt von der Absiegelung eines Kunststoffschlauches zwecks Behälterverschluss her. Die Anordnung einer Umverpackung ist nicht vorgesehen.

Als Stand der Technik sind ferner zylindrische Verpackungshülsen bekannt, die in der Regel aus zwei ineinander steckbaren Hülsenteilen bestehen, die im Innenraum einen beliebigen Gegenstand aufnehmen. Ein solcher Gegenstand kann beispielsweise ein Bohrer, ein Fräser, ein steriles medizinisches Instrument oder andere beliebige Werkzeuge sein, die gegen Stoß und Schlag optimal geschützt werden sollen.

Es ist bekannt, eine derartige, in der Regel zweiteilige Verpackungshülse dergestalt auszubilden, dass sie etwa rundzylindrisch ausgebildet ist und die Enden der beiden Verpackungshülsen, die jeweils in entgegengesetzte Richtungen weisen, scheibenförmig ausgebildet sind. Daraus ergibt sich, dass Verpackungshülsen, die zum Stand der Technik gehören, stirnseitig flach und scheibenförmig sind. Die Ebene des scheibenförmigen, stirnseitigen Abschlusses ist senkrecht zur Längsmittenachse der Verpackungshülse.

Es handelt sich demnach um stumpfe, stirnseitige Enden einer bekannten Verpackungshülse, die jedoch bei der Einbringung in eine Umverpackung, die nach der Einbringung der Verpackungshülse dann mit einem Vakuum versehen wird, schwere Nachteile erbringen.

Wenn eine solche Verpackungshülse in einer Umverpackung unter mindestens teilweisen Entzug der Verpackungsluft eingeschweißt wird, bilden sich in der Umverpackung an den stumpfendigen Stirnseiten in unerwünschter Weise Falten, welche die Lebensdauer und die Dichtheit der Verpackung reduzieren.

Es hat sich herausgestellt, dass die unerwünschte Faltenbildung in der Umverpackung zu einer Versprödung des Folienmaterials der Schlauch- oder Blattfolie im Faltenbereich führt, wenn die genannten Teile geknickt werden, wodurch durch die Faltenbildung Sollbruchstellen in der Folie gebildet werden, die bei länger dauernder Benutzung zu einem Bruch der Umverpackung und somit zu der Aufhebung des Schutzes und der Sterilität der in der Umverpackung aufgenommenen Verpackungshülse führt.

Die Erfindung ist bevorzugt für die sterile Verpackung von medizinischen Operationsinstrumenten, Werkzeugen und Implantaten vorgesehen und geeignet. Hier bestehen besondere Voraussetzungen für die Qualität der Versiegelung, weil Lufteinschlüsse im Bereich der Umverpackung an den Verpackungsenden (auch "Überstand" genannt) mit hoher Sicherheit vermieden werden müssen. Bei der Verpackung von unsterilen Lebensmitteln oder anderen Gegenständen des Alltages spielt hingegen eine solche Faltenbildung keine Rolle.

Bei der sterilen Verpackung von Operationsinstrumenten und für die Operation notwendige Gegenstände, wie Schrauben, Schienen, künstliche Hüft- oder Kniegelenke oder andere Implantate und Werkzeuge wurde jedoch festgestellt, dass die unerwünschte Faltenbildung an den Stirnseiten der Verpackung bei einer längeren Lagerzeit zu einer Versprödung der - die unerwünschte Faltungen aufweisenden - Siegelbereiche der Umverpackung führte.

Außerdem ist es üblich, die so hergestellten Verpackungen in einer weiteren Umverpackung, wie z.B. einem Karton zu lagern, wobei jedoch die stirnseitigen gesiegelten Überstände der Umverpackung zwecks besserer Verpackung abgeknickt werden.

Dieses Abknicken führt zu u einer Beschädigung der Umverpackung im Bereich der Überstände, wenn dort unerwünschte Falten vorhanden sind, was bei einer Verpackung für medizinische Gegenstände auf jeden Fall vermieden werden muss.

Dabei ist zu berücksichtigen, dass solche Umverpackungen und Verpackungsgegenstände nach der vorliegenden Erfindung mindestens für einen Zeitraum von 7 oder 8 Jahren haltbar sein müssen, ohne dass die Gefahr besteht, dass ein Bruch oder ein unerwünschtes Auftrennen der Verpackungsenden stattfindet. Die vorher erwähnte Faltenbildung führt jedoch zu einer Versprödung des Materials, und daher ist die Lebensdauer einer solchen bekannten Verpackung eingeschränkt.

Der Gegenstand der DE10301449A1 zeigt eine gesiegelte Umverpackung für einen medizinischen Gegenstand, ohne Verwendung einer Verpackungshülse und mit Darstellung der nach der Erfindung zu vermeidenden, gesiegelten Endbereiche, die eine erhebliche Faltenbildung zeigen.

Die DE 10 2007 047623 A1 offenbart eine Schutzverpackung und eine Endverpackung für Medizinprodukte. Die im inneren der Endverpackung angeordneten Gegenstände werden nach dem Verschließen der Endverpackung und dem einmaligen Sterilisieren in dem Verpackungsraum steril aufbewahrt. Die Form der Verpackung unterscheidet sich jedoch von der Form der Endverpackung, so dass sich Falten bei der folienartigen Endverpackung bilden.

Die US20120124943 A1 und die US4603538A1 zeigen eine Verpackung für medizinische Gegenstände, die aus einem äußeren gesiegelten Verpackungsbeutel besteht, in dessen Innenraum ein zweiter gesiegelter Verpackungsbeutel angeordnet ist, in dem der medizinische Gegenstand eingebracht ist. Es fehlen jedoch eine wenig biegbare, mechanisch stabile Verpackungshülse für die Aufbewahrung eines medizinischen Gegenstandes und auch die luftfreie Umschließung des inneren Beutels durch den äußeren Beutel. Die luftgefüllten Räume, welche durch die beiden Verpackungsbeutel gebildet sind, sollten vermieden werden. Eine solche Verpackung ist nicht für die Verpackung scharfkantiger Gegenstände geeignet. Der verpackte Gegenstand wird in einer solchen Verpackung auch nicht lagengesichert fixiert.

Die US20080116106A1 zeigt eine aus Kunststoff bestehende, tiefgezogene einseitig nach oben geöffnete Verpackungsschale (Tray) für die Aufnahme von medizinischen Gegenständen in der Art eines Verpackungsset, deren Öffnung mit einer Deckfolie umlaufend versiegelt ist. Damit ist keine individuelle, für jeden medizinischen Gegenstand vorhandene Verpackungshülse gezeigt, die von einer siegelfähigen Umverpackung geschlossen ist. Beim Öffnen der Deckfolie sind alle verpackten Gegenstände nicht mehr steril. Es ist bekannt, eine solche Verpackung unter Vakuum in einem sterilen Umverpackungsbeutel einzuschließen, wodurch eine doppelte Sterilität erreicht wird. Nachteil dieser Anordnung ist jedoch, dass die innere Verpackung bestehend aus der scharfkantigen Kunststoffschale beim Transport dazu neigt, durch den Umverpackungsbeutel hindurch zu stoßen, wodurch die Sterilität im äußeren Beutel verloren geht. Es besteht der weitere Nachteil, dass bei der Anbringung eines schlauchartigen Beutels, der unter Vakuum geschlossen und versiegelt wird, eine unerwünschten Faltenbildung im Bereich der Verpackungsenden entsteht, weil der Umriss der kantigen Verpackungsschale nicht an den gesiegelten Überstand angepasst ist.

Die EP 2 108 381 A1 zeigt in der gleich Weise eine aus Kunststoff bestehende, tiefgezogene Verpackungsschale, die von zwei, einen gegenseitigen Abstand zueinander aufweisenden Deckfolien abgedeckt ist, in deren Zwischenraum ein erster medizinischer Gegenstand aufgenommen ist, wobei in der Verpackungsschale der zweite Gegenstand eingelegt ist. Die beiden Deckfolien sind ringsum laufend auf den Trägerrand der Verpackungsschale aufgesiegelt. Eine individuelle Verpackungshülse für einen einzelnen Gegenstand, der dort steril verpackt ist und lagengesichert gegen Bruch geschützt ist, ist daraus nicht zu entnehmen. Eine solche Verpackung ist deshalb nur für die Verpackung von weichen, keine Kanten aufweisenden medizinischen Gegenständen, wie z.B. Schläuche geeignet, Scharfkantige und/oder schwere Operationswerkzeuge, Implantate und Werkzeuge können jedoch nicht auf diese Weise steril verpackt werden.

Aus diesem Grund hat sich die Erfindung zum Ziel gesetzt, die Lebensdauer von mit siegelfähigen Umverpackungen formschlüssig verpackten Verpackungshülsen wesentlich zu steigern und eine Faltenbildung im Bereich der gesiegelten Stirnseiten zu vermeiden.

Insbesondere soll die Lebensdauer von steril verpackten Gegenständen in einer Verpackungshülse, die formschlüssig von einer gesiegelten Umverpackung umgeben ist, wesentlich erhöht werden.

Der Erfindung liegt deshalb die Aufgabe zugrunde, eine Verpackungshülse mit einer vakumierten Umverpackung so weiterzubilden, dass beim Anlegen von Vakuum in der Umverpackung eine Faltenbildung an den Stirnseiten der Verpackungshülse weitgehend vermieden wird.

Zur Lösung der gestellten Aufgabe ist die Erfindung durch die technische Lehre des Anspruches 1 gekennzeichnet.

Eine bevorzugte Ausführungsform sieht eine medizinische Verpackung für die sterile Aufbewahrung von medizinischen Gegenständen in einer mindestens aus zwei Teilen bestehenden, luftdicht verschließbaren, sterilen Verpackungshülse mit einer mindestens teilweise formschlüssig die Verpackungshülse umschließenden, gesiegelten, folienartigen Umverpackung vor, wobei in der Umverpackung ein gegenüber der Atmosphäre verminderter Unterdruck besteht,
1. wobei mindestens eine der Stirnseiten der Verpackungshülse (1; 28, 29) abgeflacht ausgebildet ist,
2. wobei die Länge der Umverpackung (4) länger ist als die Länge der Verpackungshülse und den Stirnseitenbereich der Verpackungshülse (1, 28, 29) überragt und dort einen Überstand (10) bildet
3. wobei mindestens im Stirnseitenbereich der Verpackungshülse (1, 28, 29) die Siegel- oder Schweißnaht (5, 15) angeordnet ist,
4. wobei mindestens eine der Stirnseiten der Verpackungshülse (1; 28, 29), vorzugsweise jedoch beide Stirnseiten ein stromlinienförmiges Profil ausbilden, das von dem größeren Umfang der Verpackungshülse ausgehend in den schmalen und in der Dicke verminderten gesiegelten Überstand übergeht
5. wobei der stirnseitige Übergang von der Verpackungshülse in die Umverpackung im Wesentlichen faltenfrei ist.

In einer bevorzugten Ausgestaltung ist vorgesehen, dass mindestens eine der Stirnseiten der Verpackungshülse, vorzugsweise jedoch beide Stirnseiten abgeflacht ausgebildet sind, und von der im Durchmesser größeren Verpackungshülse in den flachen gesiegelten Überstand der Umverpackung faltenfrei übergeht.

Vorteilhaftes Merkmal ist, dass nunmehr die Stirnseiten der Verpackungshülse stromlinienförmig abgeflacht ausgebildet sind, insbesondere schnabelförmig oder spitzkeglig ausgebildet sind und dadurch die Umverpackung, welche die neuartige Verpackungshülse allseitig formschlüssig umgibt, eine faltenfreie stirnseitige Versiegelung der Verpackungshülse ermöglicht.

Damit wird ein annähernd stetiger Übergang von den im Durchmesser vergrößerten Stirnseiten der Verpackungshülse in die formschlüssig daran anschliessenden schmalen und in der Dicke verminderten Bereiche der Umverpackung im Bereich des gesiegelten Überstands erreicht, weil die flach auslaufenden Stirnseiten der Verpackungshülse an den flach ausgebildeten, gesiegelten Überstand der Umverpackung angepasst sind.

Wegen dieser harmonischen, gegenseitigen Anpassung werden kleine scharfkantige Falten und Verwerfungen im gesiegelten Überstand der Umverpackung vermieden. Es sind vielmehr große, ineinander fließende Übergangsradien im Übergangsbereich der Verpackungshülse in den umbeutelseitigen Überstand vorhanden, was durch das in axialer Richtung auslaufende, konisch in der Dicke abnehmendes, stromlinienförmige Profil der Stirnseiten der Verpackungshülse erreicht wird.

Als analoges Beispiel aus der Strömungsmechanik fließender Flüssigkeiten könnte man sagen, dass der Übergang von der Stirnseite der Verpackungshülse in den stirnseitig anschliessenden, gesiegelten Überstand der Umbeutelfolie "stromlinienförmig" gestaltet sein soll. Aus diesem Grund bilden die Stirnseiten der Verpackungshülse ein stromlinienförmiges Profil, das von der Umverpackung faltenfrei und formschlüssig umschlossen ist.

Der mindestens auf einer Stirnseite - bevorzugt jedoch auf beiden Stirnseiten - angeordnete Versiegelungsbereich der Umverpackung umschließt somit den abgeflachten Stirnseitenbereich der Verpackungshülse harmonisch, stetig und fließend und faltenfrei.

Er ist bevorzugt annähernd flach ausgebildet, weil sich die siegelfähige Umverpackung formschlüssig, nahtlos und faltenfrei an die Stirnseiten der Verpackungshülse anlegt, sodass vorteilhaft eine faltenfreie Versiegelung der Umverpackung im Vakuum erfolgen kann, wodurch Lufteinschlüsse und eine unerwünschte Faltenbildung vermieden werden.

Das bedeutet, dass ausgehend von einem annähernd rundzylindrischen oder einem daran angenäherten Querschnitt der Verpackungshülse, der im übrigen Längsbereich vorherrscht, die beiden gegenüber liegenden Stirnseiten der jeweiligen Hülsenteile nun nicht mehr rundzylindrisch ausgebildet sind, sondern abgeflacht. In einer bevorzugten Ausgestaltung ist die Abflachung etwa schnabelförmig ausgebildet. Es wird bevorzugt, wenn die Umverpackung in diesem stirnseitigen Bereich der Verpackungshülse durch eine A- oder V-förmige Versiegelung verschlossen wird.

In einer ersten Ausführungsform einer solchen Verpackungshülse handelt es sich um ein - ein- oder beidseitiges - schnabelförmiges Ende, welches in seiner Breite etwa dem Durchmesser der ansonsten (annähernd) zylinderförmigen Verpackungshülse entspricht, wobei das schnabelförmige Ende in der um 90° gedrehten Ansicht ebenfalls spitz zuläuft, um so ein Flachende zu bilden, welches nunmehr bei der Vakumierung in einer Umverpackung nicht mehr zu einer Faltenbildung der Umverpackung in diesem Bereich führt, weil es sich harmonisch fließend an den gesiegelten, flachen Überstand der Umverpackung im Stirnseitenbereich anschließt.

Erfindungsgemäß ist deshalb vorgesehen, dass die einander entgegengesetzt angeordneten Stirnseiten einer im Übrigen zylindrischen oder annähernd zylindrischen Verpackungshülse, die bevorzugt aus zwei ineinander steckbaren oder schraubbaren Hülsenteilen besteht, nunmehr als Flachenden ausgebildet sind.

Statt zweier, etwa gleichartiger Hülsenteile ist es in einer anderen Ausführung vorgesehen, dass ein einziges Hülsenteil verwendet wird, das an einer Stirnseite durch einen steck- oder schraubbaren Propfen verschlossen ist.

Ebenso ist es in einer weiteren Ausführung vorgesehen, das Hülsenteil beidseitig offen zu gestalten und jede der offenen Stirnseiten durch einen steck- oder schraubbaren Stopfen zu verschließen.

Auch in diesem Fall ist dann die Formgebung des stirnseitigen Propfens an den gewünschten harmonischen und stetigen Übergang in den gesiegelten oder verschweißten Überstand der Umverpackung anzupassen. Das bedeutet, der Propfen ist flach und/oder schnabelförmige ausgebildet, um so den faltenfreien Übergang in die Umverpackung zu gewährleisten.

Die Verpackungshülse besteht bevorzugt aus einem transparenten KunststoffMaterial, das eine solche Biegesteifigkeit aufweist, dass der eine Verpackungsteil knickfest auf den anderen Verpackungsteil zwecks Verschluß der Verpackung geschoben werden kann. Die Biegesteifigkeit soll auch so hoch sein, dass der in der Verpackung enthaltene Gegenstand gegen Bruch beim Herunterfallen der Verpackung geschützt ist und die Verpackung selbst nicht beschädigt wird.

Unter dem Begriff der "Umverpackung" versteht die Erfindung auch eine Verbundfolie in der Art einer ein- oder mehrschichtigen Verpackungsfolie, aus der flexible Verpackungen gemacht werden. Die einzelnen Folienschichten werden üblicherweise extrudiert oder kaschiert bzw. laminiert. Die Verpackungen finden hauptsächlich in der Lebensmittelindustrie Verwendung. Der Aufbau dieser Verbundfolie besteht aus einzelnen Schichten, welche für den jeweiligen Anwendungsfall spezifiziert werden. Durch eine lösemittelfreie oder auch lösemittelhaltige Kleberkaschierung erfolgt der Aufbau der einzelnen Mono-Folien zum Verbund. Auch Extrusionskaschierung oder auch Extrusionsbeschichtung - wenn auch seltener heutzutage - kommt noch zum Einsatz.

Die Trägerfolie ist die Folienschicht, welche für die Bedruckung der Folie herangezogen wird. Die Verbundfolie kann mit einer zusätzlichen Barriereschicht ausgestattet werden, wenn die Trägerfolie nicht schon genügend Eigenbarriere aufweist. Die zusätzliche Barriereschicht kann dann in die Siegelfolie eingebracht werden. Üblicherweise wird dafür EVOH - eingebettet in PE - verwendet.

Trägerfolien sind auch mit kleiner, mittlerer und höheren Barriere ausstattbar. Bei einem 2-Schicht Verbund spricht man von Duplex bei 3-Schicht von Triplex Verbundfolien. Die kaschierten Verbundfolien werden beim Thermoformen als Oberfolie und seltener als Unterfolie eingesetzt. Auch als Schalensiegelfolie für sogenannte Tray-Sealer (versiegeltes Tablett oder Schale) werden Verbundfolien eingesetzt. Verbundfolien für Schlauchbeutelmaschinen - horizontal und vertikal erfüllen schon seit vielen Jahren ihren Einsatzzweck und zwar als Schlauchbeutelfolien.

Die Verbundfolien haben je nach Träger verschiedene Eigenschaften. Die Folien können bestehen aus:
Polyester
Zellglas
Aluminium als Barriereschicht (undurchlässige Schicht)
hydrophobiertem Papier als Innenseite von Buttereinwickelfolie
PA
PE
PP
PETG

Die Erfindung betrifft auch metallisierte Folien, die z.B. auf der Basis von Polypropylen entstehen, indem im Vakuum eine Trägerfolie mit einer sehr dünnen Schicht (Reinst-)Aluminium bedampft wird. Diesem Prozess liegt die physikalische Gasphasenabscheidung zugrunde. Dadurch erhalten die Folien einen metallischen Glanz. Sie lassen sich gegen andere Folien kaschieren. Diese Behandlung geschieht nicht zuletzt auch des optischen Effektes wegen. Zum anderen bietet die metallisierte Variante Schutz vor Licht und Sauerstoff.

Unter dem Begriff "Umverpackung" versteht die Erfindung auch eine Blasfolie, Darunter versteht man Folien aus thermoplastischen Kunststoffen, welche mit Hilfe einer Blasfolienanlage gefertigt werden.

Bei Blasfolien ist die Weiterreißfestigkeit in Längs- und Querrichtung sehr ähnlich. Es gibt Folien, die nur aus einer Schicht (sog. Mono-Blasfolie) bestehen und Folien, die aus mehreren Schichten (sog. Coextrusionsblasfolie) gefertigt werden. Bei einer Coex-Blasfolie besteht die Möglichkeit, die positiven Eigenschaften verschiedener Materialien in einer Folie zu vereinen. So besteht beispielsweise eine Verpackung von Schinken aus 5 verschiedenen Schichten:
1. Außen: eine bedruckbare Schicht, meistens Polypropylen oder Polyethylen
2. Zwischen Außen und Mitte: Haftvermittler aus Ethylenvinylalkohol oder Ethylenvinylacetat,
3. Mitte: eine Sperrschicht aus Polyamid, zum "Einsperren" des Aromas
4. zwischen Mitte und Innen: Haftvermittler
5. Innen: eine lebensmittelechte Schicht mit guten Siegeleigenschaften als Sterilisierbarriere, um die Folie mit dem Unterteil der Verpackung verschweißen zu können, meistens aus Polyethylen.

Unter dem Begriff "Flachende" werden sämtliche geometrische Formen verstanden, die sich dadurch auszeichnen, dass deren Breite geringer ist als deren Höhe und die möglicherweise auch von der lichten Weite des zylindrischen Hülsenteils ausgehend, in Längsrichtung gesehen, distal spitz auslaufen. Sie können symmetrisch oder asymmetrisch zur Längsmittenachse ausgebildet sein.

Die Erfindung ist im Übrigen nicht auf zylindrische Hülsenteile der Verpackungshülse beschränkt, sondern es können auch andere Querschnitte der Hülsenteile vorgesehen werden, wie z. B. ein Quadratquerschnitt, einen ovalen Querschnitt oder ein polygonales Profil, wie z. B. ein hexagonales Profil oder dergleichen mehr.

Auch bei diesen Querschnittsformen, der miteinander zu steckenden oder zu verschraubenden Hülsenteilen, ist vorgesehen, dass die einander entgegengesetzt angeordneten Stirnseiten der Hülsenteile nunmehr als Flachenden ausgebildet sind und im Vakuum einer folienartigen Umverpackung so integriert sind, dass im Bereich dieser Stirnseiten eine Faltenbildung der Umverpackung vermieden wird.

Damit wird mit großer Sicherheit eine Vermeidung von Lufteinschlüssen im Innenraum der Umverpackung vermieden und außerdem erfolgt eine Vermeidung einer Faltenbildung, so dass auch die Umverpackung in diesen Stirnseitenbereich der Verpackungshülse vollkommen faltenfrei ist.

Damit wird die Lebensdauer einer solchen Umverpackung mit einer darin luftdicht eingeschlossenen Verpackungshülse wesentlich erhöht, weil bei Knickversuchen oder beim Abknicken der nunmehr dicht aneinander anliegenden endseitigen Enden der Umverpackung keine Falten mehr vorhanden sind und daher auch keine Bruchgefahr mehr im Bereich solcher Falten gegeben ist.

Die Erfindung ist nicht auf eine zweiteilige Verpackungshülse mit zwei ineinander steckbaren oder schraubbaren Hülsenteilen beschränkt. Es können auch mehr als zwei Hülsenteile vorhanden sein, und ebenfalls kann es vorgesehen sein, dass ein einziges Hülsenteil vorhanden ist, welches mit einem hülsenteilähnlichen Deckel oder Verschluss verschlossen wird, der erfindungsgemäß ausgebildet ist.

Ein solcher Verschluss kann dabei aufschraubbar oder aufsteckbar sein. Bei der Verpackung von sterilen Operationsinstrumenten und anderen sterilen Gegenständen hat es sich als besonders vorteilhaft herausgestellt, wenn die beiden Hülsenteile im Bereich ihrer Steck- oder Schraubverbindung nunmehr luftdicht ausgebildet sind und damit eine erste Steril-Barriere bilden.

Die zweite Steril-Barriere wird durch die vakumierte Umverpackung gebildet, die bevorzugt als Schlauchfolie ausgebildet ist, die an ihren Stirnseiten jeweils durch Schweißnähte verschlossen ist.

Bei der Auswahl der Schlauchverpackung oder der Schlauchfolie wird es bevorzugt, wenn die verschweißten Enden der Schlauchfolie an den beiden Stirnseiten auch wieder aufpeelbar sind, was bedeutet, dass die Schlauchfolie bevorzugt an ihrer Innenseite mit einer Beschichtung versehen ist, um beim Anbringen einer Siegel- oder Schweißnaht dafür zu sorgen, dass die Siegel- oder Schweißnaht auch wieder geöffnet werden kann, ohne dass die Schlauchfolie einreißt oder in anderer Weise beschädigt wird.

Auf diese Weise kann durch die Anbringung von Schnittlinien im Bereich der stirnseitigen Enden der Umverpackung dafür gesorgt werden, dass zwei voneinander trennbare, flächenförmige Folienenden ausgebildet sind, und wenn diese auseinandergezogen werden, wird die Siegel- oder Schweißnaht aufgetrennt, ohne dass die Folienenden abreißen oder einreißen.

Der Unterschied zwischen einer Siegelnaht und einer Schweißnaht ist, dass eine Siegelnaht mit einem heiß-schmelzbaren Kleber oder nur einem teilweisen Folienverbund zwischen der gesiegelten Folien herstellbar ist, während eine Schweißnaht eine stoffschlüssige Verbindung zwischen den beiden Materialien der Folien meint, wobei beide Materialien ineinander fließen und einen Verbund herstellen.

In einer bevorzugten Ausgestaltung der Erfindung ist es im Übrigen vorgesehen, dass an dem aufreißbaren Ende der Umverpackung die Siegel- oder Schweißnaht etwa spitzwinklig ausgebildet ist, d. h. sie ist nicht quer über die Breite der Umverpackung ausgerichtet, sondern sie ist etwa in der Form des Buchstabens A oder V ausgebildet, was heißt, dass die erste Aufreißbewegung an der mittigen Spitze der Siegel- oder Schweißnaht erfolgt, was mit einer relativ kleinen Aufreißkraft erfolgen kann, denn diese muss nur so groß sein, um die Spitze der A- oder V-förmigen Siegel- oder Schweißnaht zu öffnen. Ist die Spitze der Siegel- oder Schweißnaht geöffnet, kann das flache Ende der Verpackungshülse bereits schon mit der Hand heraus gezogen werden, ohne die gesamte Siegel- oder Schweißnaht öffnen zu müssen. Erst wenn eine größere Trennkraft aufgewendet wird, werden auch die seitlich an der Spitze anschließenden Schenkel der A- oder V-förmigen Siegel- oder Schweißnaht aufgetrennt und die Entnahmeöffnung in der Umverpackung wird vollständig frei, sodass die Verpackungshülse leicht entnommen werden kann.

Dabei wird es bevorzugt, wenn sich die beiden konischen Schenkel der A- oder V-förmigen Siegel- oder Schweißnaht der Umverpackung im Bereich des Überstands bis über die Seitenflächen der Verpackungshülse erstrecken, weil in diesem Fall - bei vollständiger Auftrennung der Siegel- oder Schweißnaht eine besonders gute Zugänglichkeit der Verpackungshülse zwecks Entnahme erreicht wird.

Eine Sterilität des Innenraums der Verpackungshülse wird bevorzugt durch eine Behandlung mit Gammastrahlen erreicht. In einer Weiterbildung der Erfindung ist es vorgesehen, dass sowohl die Verpackungshülse als auch deren Umverpackung steril ausgebildet sind, was dadurch erreicht wird, dass die vollständig fertig gestellte Verpackung bevorzugt mit Gammastrahlen bestrahlt wird. Es sind jedoch auch chemische Reagenzien zur Herstellung der Sterilität bekannt und werden auch hier angewendet.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, könnten als erfindungswesentlich beansprucht werden, soweit sie einzeln oder in Kombination gegenüber dem Stand der Technik neu sind. Die Verwendung der Begriffe "wesentlich" oder "erfindungsgemäß" oder "erfindungswesentlich" ist subjektiv und impliziert nicht, dass die so benannten Merkmale zwangsläufig Bestandteil eines oder mehrerer Patentansprüche sein müssen.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: eine Verpackungshülse mit vakumierter Umverpackung nach dem Stand der Technik mit einer unerwünschten Faltenbildung
- Figur 2:: Eine Verpackung in einer angepassten Schlauchfolie nach dem Stand der Technik
- Figur 3:: rechte untere Darstellung ist ein Querschnitt durch die Ausführung nach Figur 2 (Erfindung) Alle weiteren Profilformen betreffen mögliche Profile des Behälters 1 oder des Produkts 2.
- Figur 4:: die vergrößerte Darstellung des Anschlussbereiches zwischen der inneren Verpackungshülse und der Umverpackung im Stirnseitenbereich
- Figur 5:: die um 90° gedrehte Darstellung nach Figur 4 (als Stand der Technik)
- Figur 6:: die Umverpackung nach dem Stand der Technik mit einer dort eingebrachten Verpackungshülse mit Darstellung einer unerwünschten Faltenbildung
- Figur 7:: die Draufsicht einer erfindungsgemäßen Verpackungshülse mit vakumierter Umverpackung in Draufsicht
- Figur 8:: die Draufsicht gemäß Figur 7 mit einer Gesamtdarstellung der Verpackung.
- Figur 9:: die Darstellung einer Verpackungshülse, wobei der obere Teil in Draufsicht und der untere Teil in Seitenansicht dargestellt sind
- Figur 10:: die vergrößerte Darstellung von Figur 8 mit Darstellung weiterer Einzelheiten bei verbessertem Knickverhalten
- Figur 11:: die Darstellung wie Figur 10 beim Aufpeelen der beiden Folienenden, wobei eine Fingerdruckhilfe zum verbesserten Entnehmen dargestellt ist
- Figur 12:: die um 90° gedrehte Lage der Verpackung im Vergleich zur Figur 11
- Figur 13:: die Darstellung der Flachenden der Verpackungshülse in Draufsicht (obere Reihe) und im Schnitt (untere Reihe)
- Figur 14:: die Darstellung der Schlauchfolie mit Anbringung von Schnittlinien zwecks verbessertem Aufpeelen
- Figur 15:: ein Ausführungsbeispiel einer Verpackungshülse mit vakumierter Umverpackung beim Aufpeelen des Folienendes
- Figur 16:: die fortschreitende Entpackung der Verpackungshülse nach Figur 15 beim Herausdrücken aus der Umverpackung
- Figur 17:: die um 90° gedrehte Darstellung im Vergleich zu Figur 16
- Figur 18:: die Seitenansicht eines Flachendes in symmetrischer Ausführung
- Figur 19:: die Draufsicht einer um 90 Grad gedrehten Ausführung nach Figur 18
- Figur 20:: die Seitenansicht eines asymmetrischen Flachendes in drei verschiedenen Ausführungsformen
- Figur 21:: die Draufsicht der um 90 Grad gedrehten Darstellung nach Figur 20 in lediglich einer Ausführungsform
- Figur 22:: Darstellung des stromlinienförmigen Übergangsprofils der Verpackungshülse

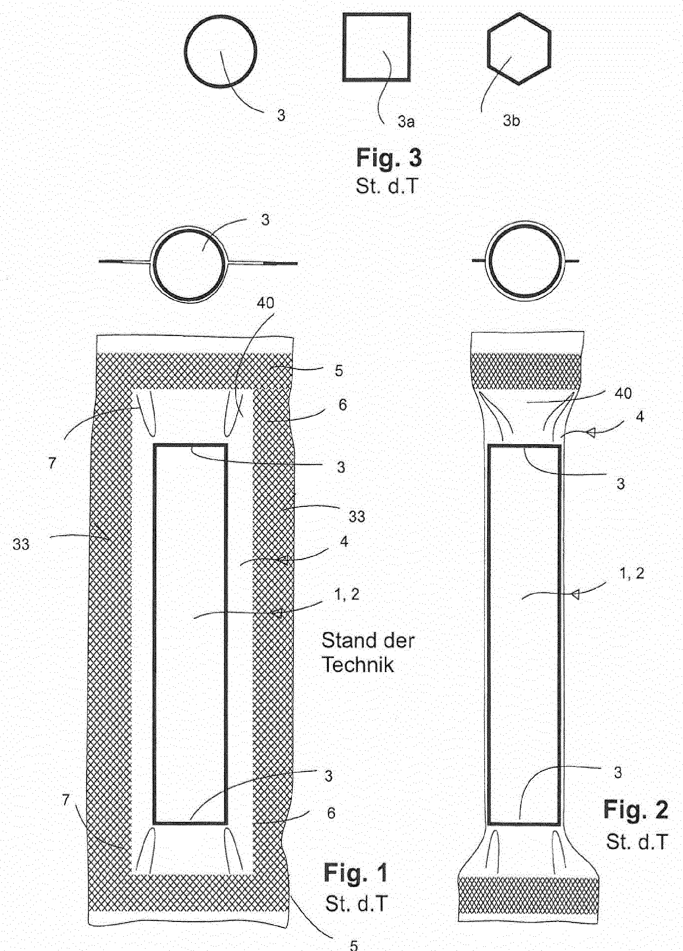

Die Figuren 1 zeigt den Stand der Technik einer Verpackungshülse, die in einer vakumierten Umverpackung eingeschlossen ist.

Die Figuren 1 zeigt einen rundzylindrischen Behälter 1, der jedoch nach der Figur 3 auch beliebige Querschnitte aufweisen kann, z. B. einen Rundquerschnitt, einen Quadratquerschnitt oder einen polygonalen Querschnitt, wie es mit dem Bezugszeichen 3, 3a und 3b dargestellt ist.

Die Figur 1 zeigt ebenfalls im Schnitt, wie beim Anlegen eines Vakuums an der Umverpackung 4 sich in unerwünschter Weise insbesondere an den Stirnseiten des Behälters 1 Falten 7 und Lufteinschlüsse 40 bilden, die zu vermeiden sind, weil sie Sollbruchstellen der Umverpackung bei länger dauernder Benutzung, Lagerung oder Transport bilden. Die erste Steriliätsbarriere kann damit durchbrochen sein.

Im gezeigten Ausführungsbeispiel nach dem Stand der Technik nach Figur 1 ist die Umverpackung 4 durch obere und untere Schweißnähte 5 luftdicht abgeschlossen.

Es ist in gestrichelten Linien dargestellt, dass jeweils auch noch Schweißnähte 33 längsseitig angeordnet sein können, so dass die Umverpackung 4 nach dem Stand der Technik und ebenso nach der Erfindung nicht nur als Schlauchfolie ausgebildet sein muss, sondern sie kann auch aus zwei zueinander parallelen Bahnen bestehen, die jeweils längsseitig durch die dargestellten Schweißnähte 5, 33 verbunden sind.

Im Innenraum des Behälters 1 ist ein beliebiges Produkt 2 angeordnet, welches - entsprechend der allgemeinen Beschreibung - beispielsweise als Werkzeug oder als anderer stückiger Gegenstand (z.B. auch als Verpackungshülse) ausgebildet sein kann.

Wichtig ist, dass die Stirnseite 3 des Behälters 1 nach dem Stand der Technik eben und flach ist, das bedeutet, sie verjüngt sich nicht in distaler Richtung, sondern ist z. B. als rundzylindrische Scheibe oder dergleichen ausgebildet.

Bei einem Luftentzug aus dem Innenraum der Umverpackung soll sich diese möglichst formschlüssig und faltenfrei an die im Innenraum angeordnete Verpackungshülse anschmiegen.

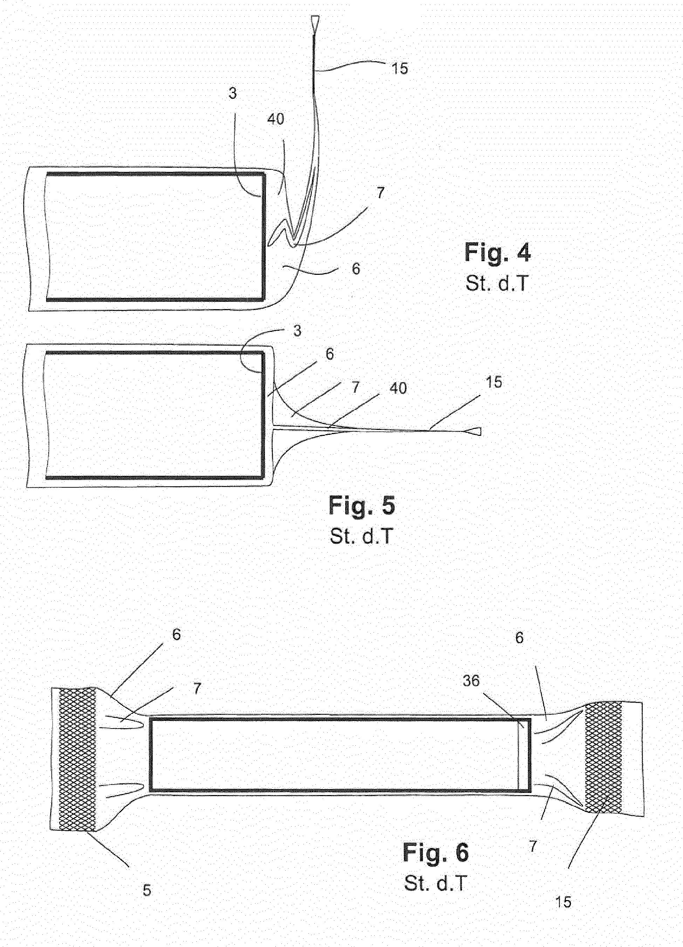

Es bildet sich damit im Bereich der Umverpackung 4 ein unerwünschter Übergangsbereich 6, in dem bei einem Luftentzug aus dem Innenraum der Umverpackung Lufteinschlüsse 40 im Bereich von Falten 7 bilden.,
Durch die Verklebung der Folienlagen der Umverpackung bei einem Luftentzug bilden sich in unerwünschter Weise Falten 7 im Stirnseitenbereich 3. Diese bilden biegesteife, versprödete Folien-Bereiche, die dann bei einer Abknickung des stirnseitigen Endes zu Bruchstellen im Übergangsbereich 6 führen, so dass die Dichtheit der Umverpackung aufgehoben wird. Dies ist in den Figuren 4 und 5 jeweils in der Seitenansicht dargestellt, wo erkennbar ist, dass neben den unerwünschten Lufteinschlüssen vor allem die unerwünschten Falten 7 ausgebildet werden, die bei einem wiederholten Abknicken zu einem Aufbrechen der Umverpackung führen.

Die Figur 6 zeigt die gleichen Teile wie die Figur 2 mit Darstellung weiterer Einzelheiten. Es kann beispielsweise vorgesehen sein, dass der Behälter 1 durchgehend ausgebildet ist und beispielsweise durch einen Deckel 36 verschlossen ist, und es kann in einer anderen Ausgestaltung vorgesehen sein, dass der Behälter 1 aus zwei miteinander verbundenen Hülsenteilen 28, 29 besteht, was anhand der späteren Erfindungsbeschreibung noch näher erläutert werden wird.

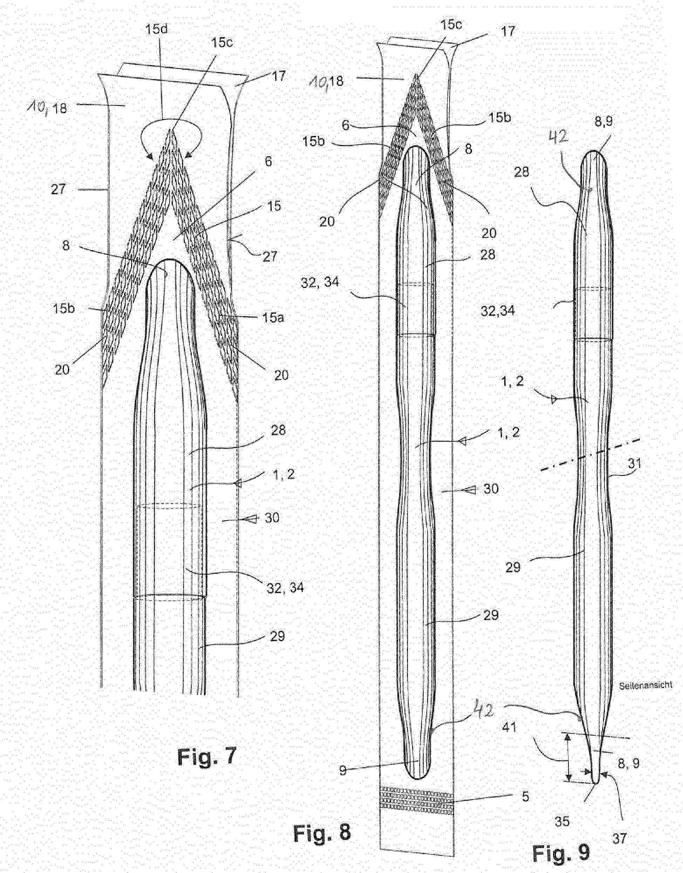

Die Figur 7 zeigt nunmehr das obere Ende einer erfindungsgemäßen Verpackungshülse mit einer vakumierten Umverpackung, wo erkennbar ist, dass der Behälter 1 nunmehr aus zwei Hülsenteilen 28, 29 besteht, die im Bereich eines Dichtverschlusses 34 über einen Steck- oder Schraubbereich 32 abgedichtet miteinander verbunden sind.

Es ist nicht dargestellt, dass der Dichtverschluss 34 im Bereich des Steck- oder Schraubbereiches 32 noch mit weiteren Dichtmaßnahmen ausgerüstet sein kann, z. B. können dort O-Ring-Dichtungen angeordnet sein. Es kann auch eine Verklebung vorhanden sein oder eine in sich luftdichte Steck- oder Schraubverbindung. Ebenso können in diesem Dichtverschluss-Bereich auch abdichtend aufgeklebte Etiketten verwendet werden und dergleichen mehr.

Wichtig ist, dass im Innenraum des Behälters 1, d. h. also im Innenraum der Hülsenteile 28, 29 eine Steril-Atmosphäre aufrechterhalten wird, weil der Dichtverschluss 34 ein Eindringen von Keimen in den Innenraum der Hülsenteile 28, 29 verhindert.

Selbstverständlich ist es in einer anderen Ausgestaltung möglich, dass die Hülsenteile 28, 29 nicht spiegelsymmetrisch gleich ausgebildet sind, sondern der eine Hülsenteil 29 kann sehr lang ausgebildet sein und der andere Hülsenteil 28 kann - in axial verkürzter Ausführung - lediglich als Schraub- oder Steckverschluss deckelartig ausgebildet sein.

Die Figur 7 zeigt eine spezielle Anordnung des erfindungsgemäßen Flachendes 8, welches die Stirnseite des Hülsenteils 28 bildet.

Es kann vorgesehen sein, dass die beiden Stirnseiten der Hülsenteile 28, 29 als Flachenden 8, 9 ausgebildet sind, und es kann in einer anderen Ausgestaltung auch nur vorgesehen sein, dass nur die Stirnseite eines Hülsenteils 28 oder 29 als Flachende 8 oder 9 ausgebildet sein kann.

Der einfacheren Beschreibung wegen wird lediglich ein einziges Flachende 8 an dem Hülsenteil 28 als erfindungsgemäß beschrieben, wobei bevorzugt - aber nicht ausschließlich - vorausgesetzt wird, dass auch das andere Hülsenteil 29 mit einem derartigen Flachende 9 ausgerüstet sein kann.

Im Ausführungsbeispiel nach Figur 7 und 8 ist eine besondere A-förmige Siegel- oder Schweißnaht 15 gezeigt, die aus insgesamt zwei im spitzen Winkel zueinander laufenden Schenkeln 15a, 15b besteht, die in einer mittleren Spitze 15c zusammenlaufen. Sie bilden einen spitzen Winkel 15d, weil die Schlauchfolie zunächst zum Aufpeelen an den eingezeichneten Schnittlinien 27 stirnseitig eingeschnitten wird, um so zwei voneinander trennbare Folienblätter 17, 18 zu erhalten. Wichtig ist, dass sich die Siegel- oder Schweißnaht 15 auch bis zu den Seitenkanten der Schlauchfolie erstreckt, um so an den beiden gegenüber liegenden Enden 20 einen dichten Abschluss zu erreichen.

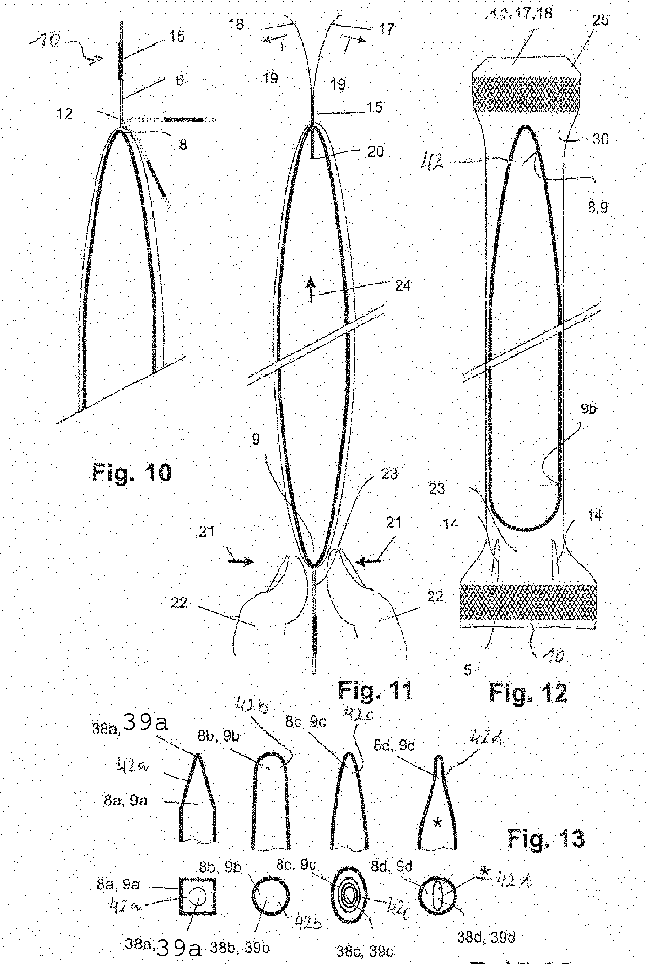

Beim Auftrennen der beiden Folienblätter 17, 18 in Pfeilrichtung 19 (siehe Figur 11) können somit die beiden Folienblätter 17, 18 voneinander getrennt werden. Die A-Form der Siegel- oder Schweißnaht 15 hat den Vorteil der leichten und kontrollierten Trennbarkeit der beiden Folienblätter 17, 18 in den eingezeichneten Pfeilrichtungen 19 (siehe Figur 11), weil die Trennkraft zunächst in schwächerer Form auf die Spitze 15c der Siegel- oder Schweißnaht 15 wirkt und dann mit stärkerem Fortschreiten der Aufreißbewegung sich in die beiden im Winkel zueinander angeordneten Schenkel 15a, 15b fortsetzt.

Mit dem Bezugszeichen 30 ist allgemein dargestellt, dass die Umverpackung 4 erfindungsgemäß als Schlauchfolie 30 ausgebildet ist.

In einer anderen Ausgestaltung kann es jedoch auch vorgesehen sein, dass statt einer Schlauchfolie zueinander parallele Folienblätter 17, 18 verwendet werden, die längsseitig durch jeweils eine Siegel- oder Schweißnaht 33 - siehe Figur 1 - gerade oder gekurvt - miteinander verbunden sind.

Wie bereits im allgemeinen Teil ausgeführt, verbinden sich die beiden Hülsenteile 28, 29 in einem Steck- oder Schraubbereich 32 und bilden dort einen Dichtverschluss 34, der dafür sorgt, dass der Innenraum des Behälters 1 vollkommen luftdicht gegenüber der Atmosphäre und gegenüber dem Innenraum der Umverpackung und der Schlauchfolie 30 abgeschlossen ist.

Im Übrigen wird in einer bevorzugten Ausgestaltung der Erfindung angegeben, dass im mittleren Bereich der beiden Hülsenteile 28, 29 eine Einschnürung 31 vorhanden ist, die für eine bessere Lagensicherung eines im Innenraum angeordneten - etwa zylindrischen - Gegenstandes, wie z. B. einen Fräser, einen Bohrer oder ein chirurgisches Instrument sorgt.

Wichtig ist gemäß der Erfindung, dass in der Draufsicht nunmehr eine oder beide Stirnseiten der beiden Hülsenteile 28, 29 als Flachenden 8, 9 ausgebildet sind.

In der Figur 9 sind zwei räumlich zueinander gedrehte Darstellungen gezeigt, d. h. der obere Teil der Figur 9 zeigt das Flachende 8 in Seitenansicht, während der untere Teil der Darstellung in Figur 9 das gleiche Hülsenteil mit einer um 90° gedrehten Ansicht zeigt, wo erkennbar ist, dass das Flachende 8, 9 längssymmetrisch spitz ausläuft und spitzenseitig einen geringen Radius 35 aufweist.

Die Dicke 37 des Flachendes ist somit wesentlich dünner als vergleichsweise der Durchmesser des Behälters 1 und beträgt etwa 1/10 des Durchmessers des Behälters 1 im übrigen Bereich.

Bei einem Behälterdurchmesser 1 von z.B. 20 mm beträgt dann die Dicke 37 des jeweiligen Flachendes 8, 9 nur noch z. B. 2 mm.
Durch das bevorzugte Hohlblasverfahren ergibt sich im Flachende eine - verfahrensbedingt - vergrößerte Wandstärke, die eine Durchdringungsbarriere für ein dort aufliegendes spitzes Werkzeug bildet.

Die axiale Länge des Flachendes kann beliebig gewählt werden. Im gezeigten Ausführungsbeispiel hat das Flachende beispielsweise eine Längserstreckung von 50 mm. Bevorzugt wird eine Länge entsprechend dem doppelten Durchmesser des Behälters 1 gewählt.

Ebenso kann es vorgesehen sein, dass die Länge 41 des Flachendes 8, 9 etwa dem Durchmesser des Behälters 1 entspricht; es kann jedoch auch das zweifache oder dreifache des Durchmessers als Länge 41 gewählt werden.

Im Ausführungsbeispiel nach Figur 8 ist auch das untere Ende der Schlauchfolie 30 als von der oberen Siegel- oder Schweißnaht 15 abweichende Siegel- oder Schweißnaht 5 dargestellt.

Deshalb ist im Ausführungsbeispiel nach Figur 7 und 8 die obere Siegel- oder Schweißnaht 15 zur Entnahme des Behälters 1 vorgesehen, während die untere Siegel- oder Schweißnaht 5 in der Regel nicht zu öffnen ist.

Die Figuren 10 und 11 zeigen, dass wegen der fehlenden Faltenbildung oder der Vermeidung einer Faltenbildung auch ein Abknicken des Folienbereiches der Umverpackung im Bereich einer Knickstelle 12 nicht zu einem Bruch der Umverpackung in diesem Bereich führt, weil dort keinerlei Falten mehr vorhanden sind.

Auch im Übergangsbereich 6, der sich in Richtung auf die Siegel- oder Schweißnaht 15 bildet, sind keinerlei Falten mehr vorhanden.

Die Figur 11 zeigt einen Entnahmevorgang einer spiegelsymmetrisch ausgebildeten Verpackungshülse gemäß Figur 10, wo erkennbar ist, dass das obere Flachende 8 genau gleich wie das untere Flachende 9 ausgebildet ist und daher ein besonders einfacher Entnahmevorgang stattfinden kann.

Wenn nämlich auf das untere Flachende 9 mit dem Druck der Finger 22 in Pfeilrichtung 21 gedrückt wird, wird das untere Flachende 9 in der Umverpackung in Pfeilrichtung 24 nach oben verschoben und das obere Flachende 8 kann dann entnommen werden, sofern die beiden Folienblätter 17, 18 in Pfeilrichtung 19 mit den Fingern einer Hand auseinander gezogen werden, so dass auch die obere Siegel- oder Schweißnaht 15 aufgetrennt wird.

Die Figur 12 zeigt ein abgewandeltes Ausführungsbeispiel, wo erkennbar ist, dass die obere Siegel- oder Schweißnaht auch als Querverschluss ausgebildet sein kann, wobei sich dann ebenfalls die voneinander trennbaren Folienblätter 17, 18 ergeben.

Es ist ebenso in Figur 12 dargestellt, dass aufgrund des stark abgerundeten Flachendes 9b nunmehr sich erlaubter maßen im unteren Schlauchendebereich 23 kleine Falten 14 ergeben, die jedoch nicht stören.

Wichtig ist jedenfalls, dass mindestens in einem Bereich der Schlauchfolie 30, d.h. also im Bereich des oberen Flachendes 8 und/oder im Bereich des unteren Flachendes 9 eine Faltenbildung vermieden wird.

Die Vermeidung einer Faltenbildung sowohl an der oberen als auch an der unteren Seite der Schlauchfolie 30 ist jedoch nicht immer zwingend notwendig.

Die Figur 13 zeigt verschiedene Ausführungsformen von Flachenden, wobei die obere Reihe die Seitenansicht unterschiedlicher Ausführungen der Flachenden 8, 9 darstellt, während die untere Reihe die dazu gehörenden Stirnansichten dieser Flachenden 8, 9 darstellt.

Es ist noch jeweils angegeben, dass die Flachenden jeweils Spitzen 38, 39 ausbilden, so dass in der unteren Darstellung jeweils auch die Draufsicht auf die Spitzen 38, 39 in den verschiedenen Ausführungsformen dargestellt ist.

In der ersten Spalte in Figur 13 ist deshalb eine relativ spitz auslaufende Spitze 38a, 39a dargestellt, und das Flachende ist etwa quadratisch ausgebildet und läuft deshalb in die Spitze 38a, 39a aus. In der zweiten Spalte der Figur 3 ist dargestellt, dass das Flachende 8b, 9b etwa schnabelförmig abgerundet ausläuft und in der Draufsicht noch ein gegenüber dem Durchmesser des Behälters 1 verminderte Spitze 38b, 39b ausbildet.

In Figur 13 ist in der dritten Spalte eine relativ spitzwinklige Ausgestaltung des Flachendes 8c, 9c dargestellt, und die dazugehörenden Spitzen 38c und 39c verlaufen genau symmetrisch, gehen jedoch von einem ovalen Querschnitt aus. In der rechten Spalte in Figur 13 ist die Spitze 38d, 39d noch weiter angespitzt und läuft im Übrigen in einen etwa ovalen Querschnitt aus. Eine solche Querschnittsform hat sich bei Versuchen als optimale Lösung heraus gestellt.

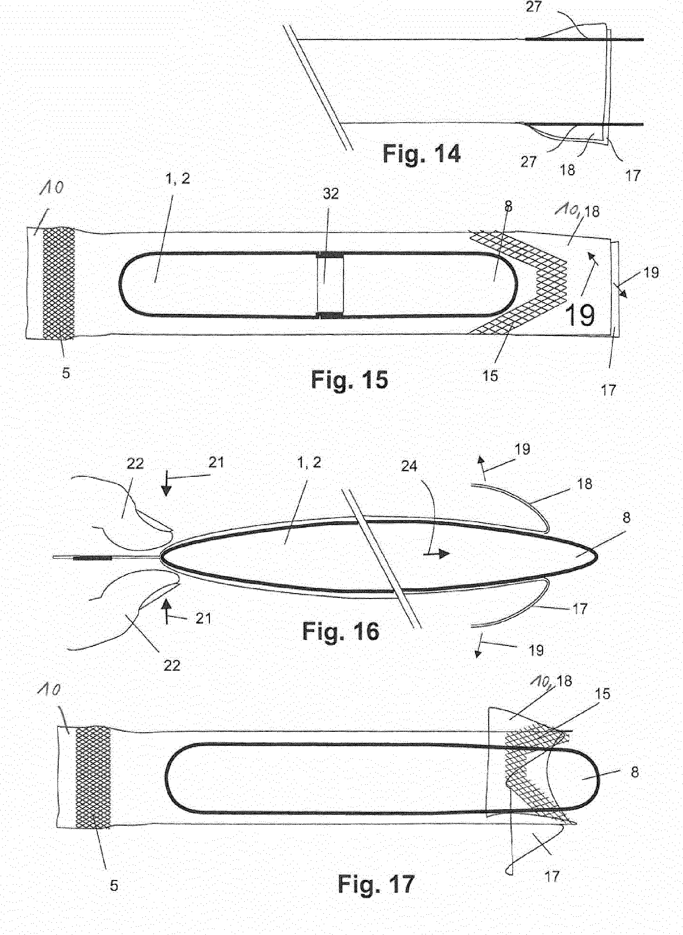

Die Figur 14 zeigt den Aufpeelvorgang, der in Figur 11 dargestellt ist, wo erkennbar ist, dass entlang von Schnittlinien 27 der Folienschlauch aufgetrennt werden kann.

Die Figuren 15 und 16 zeigen einen Entnahmevorgang eines Behälters 1 mit einem darin angeschlossenen Produkt 2 in der Art, wie er in Figur 11 bereits schon erläutert wurde, wobei in den Figuren 15 die Draufsicht und in Figur 16 die Seitenansicht dargestellt sind.

Die Figur 17 zeigt die fast vollendete Entnahmeposition, so dass nunmehr bei abgepeelten Folienblättern 17, 18 das obere Flachende 8 entnommen werden kann.

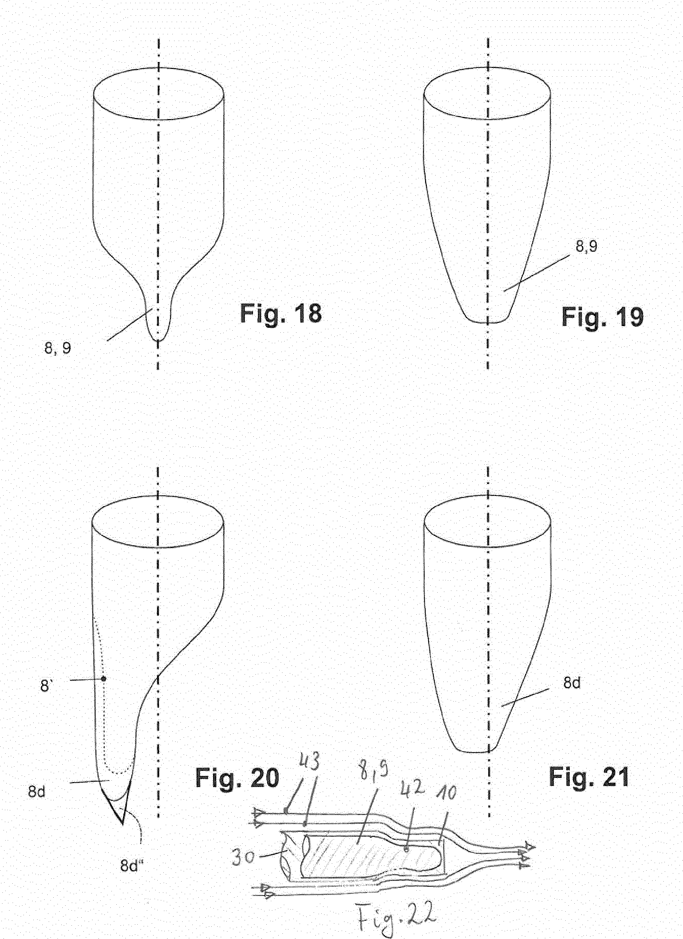

Die Figuren 18 und 19 zeigen in Übereinstimmung mit den vorherigen Ausführungsbeispielen eine spiegelsymmetrische Ausbildung der Flachenden 8, 9 in Seitenansicht und Draufsicht, während die Figuren 20 und 21 drei verschiedene Ausführungsformen von asymmetrisch ausgebildeten Flachenden zeigen.

In Figur 20 ist ein asymmetrisches Flachende 8d dargestellt, welches außermittig zur Längsmittenachse ausgebildet ist und dieses Flachende 8d kann entsprechend auch eine Formgebung 8d oder eine Formgebung 8d" einnehmen.

Die Figur 22 zeigt als Prinzipskizze, wie ein von (gas- oder flüssigkeitsförmigen) Stromlinien 43 umströmter Körper in der Form der erfindungsgemäßen Verpackungshülse das Übergangsprofil 42 ausbildet. Es ist erkennbar, dass das Übergangsprofil 42 an den Stirnseiten der Verpackungshülse etwa einer Stromlinie 43 entspricht. Das bedeutet, dass eine stetiger und harmonischer Übergang von dem größeren Querschnitt der Verpackungshülse im Mittenbereich in die abgeflachte Stirnseite der Verpackungshülse in der Art einer Stromlinie gegeben ist.

### Zeichnungslegende

- 1: Behälter
- 2: Produkt
- 3: Stirnseite
- 4: Umverpackung
- 5: Siegel- oder Schweißnaht (am Boden)
- 6: Übergangsbereich
- 7: Falte
- 8: Flachende a, b, c, d
- 9: Flachende a, b, c, d
- 10: Überstand
- 11 12: Knickstelle
- 13 14: Falte
- 15: Siegel- oder Schweißnaht (Spitze)
- 15a: Schenkel
- 15b: Schenkel
- 15c: Spitze
- 15d: Winkel
- 16 17: Folienblätter
- 18: Folienblätter
- 19: Pfeilrichtung
- 20: Ende (von 15)
- 21: Pfeilrichtung
- 22: Finger
- 23: Schlauchende
- 24: Pfeilrichtung
- 25: Schnittlinie
- 26 27: Schnittlinie
- 28: Hülsenteil
- 29: Hülsenteil
- 30: Schlauchfolie
- 31: Einschnürung
- 32: Steck- oder Schraubbereich
- 33: Schweißnaht (Stand der Technik)
- 34: Dichtverschluss
- 35: Radius
- 36: Deckel
- 37: Dicke
- 38: Spitze a, b, c, d
- 39: Spitze a, b, c, d
- 40: Lufteinschluss
- 41: Länge
- 42: Übergangsprofil (stromlinienförmig)
- 43: Stromlinie

## Patentansprüche

1. Medizinische Verpackung für die sterile Aufbewahrung von medizinischen Gegenständen in einer mindestens aus zwei Teilen bestehenden, luftdicht verschließbaren, sterilen Verpackungshülse (1; 28, 29) mit einer formschlüssig die Verpackungshülse (1; 28, 29) umschließenden, gesiegelten, folienartigen Umverpackung (4), wobei in der Umverpackung (4) ein gegenüber der Atmosphäre verminderter Unterdruck besteht und die Länge der Umverpackung (4) länger ist als die Länge der Verpackungshülse (1; 28, 29) und den Stirnseitenbereich der Verpackungshülse (1, 28, 29) überragt und dort einen axialen Überstand (10) bildet, wobei eine Siegel- oder Schweißnaht (5, 15) mindestens im Stirnseitenbereich der Verpackungshülse (1, 28, 29) angeordnet ist und mindestens eine der Stirnseiten der Verpackungshülse (1; 28, 29), vorzugsweise jedoch beide Stirnseiten ein stromlinienförmiges Profil ausbilden, das ausgehend von dem größeren Umfang der Verpackungshülse (1; 28, 29) in einen schmalen und in der Dicke verminderten Bereich übergeht, **dadurch gekennzeichnet, dass** die mindestens eine und/oder beide Stirnseiten der Hülsenteile (28, 29) der Verpackungshülse als Flachenden (8, 9) ausgebildet sind, wobei die flach auslaufenden Stirnseiten der Verpackungshülse an den flach ausgebildeten, gesiegelten Überstand (10) der Umverpackung (4) angepasst sind und dadurch der stirnseitige Übergang von der Verpackungshülse (1, 28, 29) in die Umverpackung (4) im Wesentlichen faltenfrei ist.

2. Medizinische Verpackung nach Anspruch 1, **dadurch gekennzeichnet, dass,** das stromlinienförmige Profil der mindestens einen Stirnseite der Verpackungshülse (1; 28, 29) vorzugsweise beider Stirnseiten schnabelförmig oder spitzkeglig ausgebildet ist.

3. Medizinische Verpackung nach Anspruch 1 oder 2, **dadurch gekennzeichnet, dass** ausgehend von einem annähernd rundzylindrischen oder einem daran angenäherten Querschnitt der Verpackungshülse (1; 28, 29), der im übrigen Längsbereich vorherrscht, die beiden gegenüber liegenden Stirnseiten abgeflacht und/oder etwa schnabelförmig ausgebildet sind.

4. Medizinische Verpackung nach einem der Ansprüche 1 bis 3, **dadurch gekennzeichnet, dass** das schnabelförmige Ende, welches in seiner Breite etwa dem Durchmesser der ansonsten zylinderförmigen Verpackungshülse entspricht, in der um 90° gedrehten Ansicht ebenfalls spitz zuläuft, um so ein Flachende zu bilden, welches bei der Vakuumierung in einer Umverpackung (4) nicht mehr zu einer Faltenbildung der Umverpackung in diesem Bereich führt.

5. Medizinische Verpackung nach einem der Ansprüche 1 bis 4, **dadurch gekennzeichnet, dass** sie aus mindestens zwei Hülsenteilen (28, 29) besteht, die mit einer Steck- oder Schraubverbindung (32) abgedichtet miteinander verbunden sind.

6. Medizinische Verpackung nach einem der Ansprüche 1 bis 5, **dadurch gekennzeichnet, dass** die Umverpackung (4) mindestens an einer Seite aufpeelbar ist.

7. Medizinische Verpackung nach einem der Ansprüche 1 bis 6 , **dadurch gekennzeichnet, dass** an einem aufreißbaren Ende der Umverpackung (4) die Siegel- oder Schweißnaht (15) etwa spitzwinklig in der Art einer V-Form ausgebildet ist.

8. Medizinische Verpackung nach einem der Ansprüche 1 bis 7, **dadurch gekennzeichnet, dass** die Umverpackung (4) als Schlauchfolie (3) ausgebildet ist.

9. Medizinische Verpackung nach einem der Ansprüche 1 bis 8, **dadurch gekennzeichnet, dass** das Flachende (8, 9) spiegelsymmetrisch zur Längsmittenachse des Behälters (1) ausgebildet ist.

10. Medizinische Verpackung nach einem der Ansprüche 1 bis 8, **dadurch gekennzeichnet, dass** das Flachende (8, 9) asymmetrisch zur Längsmittenachse des Behälters (1) ausgebildet ist.

11. Medizinische Verpackung nach einem der Ansprüche 1 bis 10, **dadurch gekennzeichnet, dass** die axiale Länge des Flachendes (8, 9) etwa dem doppelten Durchmesser des Behälters (1) entspricht.

12. Medizinische Verpackung nach einem der Ansprüche 1 bis 11, **dadurch gekennzeichnet, dass** die distale, spitzenseitige Dicke des Flachendes etwa 1/10 des Durchmessers des Behälters (1) entspricht.

13. Medizinische Verpackung nach einem der Ansprüche 1 bis 12, **dadurch gekennzeichnet, dass** das Flachende (8, 9) eine geradlinige Längserstreckung aufweist.

14. Medizinische Verpackung nach einem der Ansprüche 1 bis 12, **dadurch gekennzeichnet, dass** das Flachende (8, 9) eine bogenförmige Längserstreckung, insbesondere eine Löffelform, aufweist.

15. Medizinische Verpackung (1; 28, 29) nach einem der Ansprüche 1 bis 14, **dadurch gekennzeichnet, dass** mindestens der Innenraum der Verpackungshülse (1, 28, 29) steril ist.

16. Medizinische Verpackung (1; 28, 29) nach einem der Ansprüche 1 bis 15, **dadurch gekennzeichnet, dass** mindestens der vakumierte Zwischenraum zwischen der Umverpackung (4) und der Verpackungshülse (1, 28, 29) steril ausgebildet ist

## Claims

1. Medical packaging for the sterile storage of medical objects in a sterile packaging sleeve (1; 28, 29) consisting at least of two parts and sealable to be air-tight with a sealed, foil-like outer packaging (4) positively surrounding the packaging sleeve (1; 28, 29), wherein in the outer packaging (4) there is a negative pressure reduced with respect to the atmosphere and the length of the outer packaging (4) is longer than the length of the packaging sleeve (1; 28, 29) and protrudes beyond the end-face side region of the packaging sleeve (1, 28, 29) and forms an axial projecting length (10) there, wherein a sealing seam or welding seam (5, 15) is arranged at least in the end-face side region of the packaging sleeve (1, 28, 29) and at least one of the end-face sides of the packaging sleeve (1; 28, 29), but preferably both end-face sides, form a streamlined profile which, starting from the greater circumference of the packaging sleeve (1; 28, 29), changes into a thin region with reduced thickness, **characterised in that** the at least one and/or both end-face sides of the sleeve parts (28, 29) of the packaging sleeve are designed as flat ends (8, 9), wherein the end-face sides of the packaging sleeve running flat are adapted to the sealed projecting length (10) of the outer packaging (4) designed to be flat and thus the end-face side transition from the packaging sleeve (1, 28, 29) to the outer packaging (4) is substantially crease-free.

2. Medical packaging according to claim 1, **characterised in that** the streamlined profile of the at least one end-face side of the packaging sleeve (1; 28, 29), preferably of both end-face sides, is designed like a beak or pointed cone.

3. Medical packaging according to claim 1 or 2, **characterised in that**, starting from an approximately round-cylindrical cross-section of the packaging sleeve (1; 28, 29) or one approximated thereto, which prevails in the remaining longitudinal region, the two opposite end-face sides are flattened off and/or designed to be approximately like a beak.

4. Medical packaging according to one of claims 1 to 3, **characterised in that** the beak-like end, which corresponds as regards its width approximately to the diameter of the otherwise cylindrical packaging sleeve, in the view rotated by 90° likewise tapers off to thus form a flat end which no longer leads to crease formation of the outer packaging in this region during vacuum-packaging in an outer packaging (4).

5. Medical packaging according to one of claims 1 to 4, **characterised in that** it consists of at least two sleeve parts (28, 29) which are joined to one another in sealed manner using a plug connection or screw connection (32).

6. Medical packaging according to one of claims 1 to 5, **characterised in that** the outer packaging (4) can be peeled open at least on one side.

7. Medical packaging according to one of claims 1 to 6, **characterised in that** the sealing seam or welding seam (15) is designed to be approximately acute-angled like a V shape at a tearable end of the outer packaging (4).

8. Medical packaging according to one of claims 1 to 7, **characterised in that** the outer packaging (4) is designed as a tubular foil (3).

9. Medical packaging according to one of claims 1 to 8, **characterised in that** the flat end (8, 9) is designed to be mirror-symmetrical with the central longitudinal axis of the container (1).

10. Medical packaging according to one of claims 1 to 8, **characterised in that** the flat end (8, 9) is designed to be asymmetrical with the central longitudinal axis of the container (1).

11. Medical packaging according to one of claims 1 to 10, **characterised in that** the axial length of the flat end (8, 9) corresponds approximately to twice the diameter of the container (1).

12. Medical packaging according to one of claims 1 to 11, **characterised in that** the distal, point-side thickness of the flat end corresponds approximately to 1/10 of the diameter of the container (1).

13. Medical packaging according to one of claims 1 to 12, **characterised in that** the flat end (8, 9) has a straight-line longitudinal extension.

14. Medical packaging according to one of claims 1 to 12, **characterised in that** the flat end (8, 9) has a curved longitudinal extension, in particular a spoon shape.

15. Medical packaging (1; 28, 29) according to one of claims 1 to 14, **characterised in that** at least the interior of the packaging sleeve (1, 28, 29) is sterile.

16. Medical packaging (1; 28, 29) according to one of claims 1 to 15, **characterised in that** at least the evacuated space between the outer packaging (4) and the packaging sleeve (1, 28, 29) is designed to be sterile.

## Revendications

1. Emballage médical pour la conservation stérile d'objets médicaux dans un étui d'emballage stérile (1 ; 28, 29) composé d'au moins deux parties et apte à être fermé de manière étanche à l'air, avec un suremballage (4) en forme de film, scellé, entourant par complémentarité de forme l'étui d'emballage (1 ; 28, 29), dans lequel une dépression réduite par rapport à l'atmosphère est présente dans le suremballage (4) et la longueur du suremballage (4) est plus longue que la longueur de l'étui d'emballage (1 ; 28, 29) et dépasse de la zone de côtés frontaux de l'étui d'emballage (1 ; 28, 29) et forme à cet endroit une saillie axiale (10), dans lequel un joint scellé ou soudé (5, 15) est disposé au moins dans la zone de côtés frontaux de l'étui d'emballage (1 ; 28, 29) et l'un au moins des côtés frontaux de l'étui d'emballage (1 ; 28, 29), mais de préférence les deux côtés frontaux forment un profil aérodynamique qui, à partir de la plus grande circonférence de l'étui d'emballage (1 ; 28, 29), passe à une zone étroite et à épaisseur réduite, **caractérisé en ce que** le et/ou les deux côtés frontaux des parties d'étui (28, 29) de l'étui d'emballage (1 ; 28, 29) sont formés comme des extrémités plates (8, 9), dans lequel les côtés frontaux à extrémités plates de l'étui d'emballage (1 ; 28, 29) sont adaptés à la saillie (10) scellée, de forme plate, du suremballage (4) et le passage, côté frontal, de l'étui d'emballage (1 ; 28, 29) au suremballage (4) est ainsi sensiblement sans plis.

2. Emballage médical selon la revendication 1, **caractérisé en ce que** le profil aérodynamique du côté frontal de l'étui d'emballage (1 ; 28, 29), de préférence des deux côtés frontaux, est en forme de bec ou de cône pointu.

3. Emballage médical selon la revendication 1 ou 2, **caractérisé en ce qu'**à partir d'une section transversale de l'étui d'emballage (1 ; 28, 29), approximativement cylindrique ronde ou s'approchant de cette forme, prédominante dans le reste de la zone longitudinale, les deux côtés frontaux opposés sont aplatis et/ou sont à peu près en forme de bec.

4. Emballage médical selon l'une des revendications 1 à 3, **caractérisé en ce que** l'extrémité en forme de bec qui correspond par sa largeur à peu près au diamètre de l'étui d'emballage par ailleurs cylindrique, se termine également en pointe sur une vue tournée de 90°, pour former ainsi une extrémité plate qui, lors de la mise sous vide dans un suremballage (4), n'entraîne plus de formation de plis du suremballage dans cette zone.

5. Emballage médical selon l'une des revendications 1 à 4, **caractérisé en ce qu'**il se compose d'au moins deux parties d'étui (28, 29) qui sont reliées entre elles de manière étanche avec un liaison à emboîtement ou à vis (32).

6. Emballage médical selon l'une des revendications 1 à 5, **caractérisé en ce que** le suremballage (4) est pelable sur au moins un côté.

7. Emballage médical selon l'une des revendications 1 à 6, **caractérisé en ce qu'**à une extrémité déchirable du suremballage (4), le joint scellé ou soudé (15) est à angle aigu à la manière d'une forme en V.

8. Emballage médical selon l'une des revendications 1 à 7, **caractérisé en ce que** le suremballage (4) est formé comme un film tubulaire.

9. Emballage médical selon l'une des revendications 1 à 8, **caractérisé en ce que** l'extrémité plate (8, 9) est formée en miroir par rapport à l'axe longitudinal médian du récipient (1).

10. Emballage médical selon l'une des revendications 1 à 8, **caractérisé en ce que** l'extrémité plate (8, 9) est formée asymétriquement par rapport à l'axe longitudinal médian du récipient (1).

11. Emballage médical selon l'une des revendications 1 à 10, **caractérisé en ce que** la longueur axiale de l'extrémité plate (8, 9) correspond à peu près au double du diamètre du récipient (1).

12. Emballage médical selon l'une des revendications 1 à 11, **caractérisé en ce que** l'épaisseur distale, côté pointe, de l'extrémité plate correspond à peu près à 1/10 du diamètre du récipient (1).

13. Emballage médical selon l'une des revendications 1 à 12, **caractérisé en ce que** l'extrémité plate (8, 9) présente une extension longitudinale rectiligne.

14. Emballage médical selon l'une des revendications 1 à 12, **caractérisé en ce que** l'extrémité plate (8, 9) présente une extension longitudinale courbe, en particulier en forme de cuiller.

15. Emballage médical (1 ; 28, 29) selon l'une des revendications 1 à 14, **caractérisé en ce qu'**au moins l'espace intérieur de l'étui d'emballage (1 ; 28, 29) est stérile.

16. Emballage médical (1 ; 28, 29) selon l'une des revendications 1 à 15, **caractérisé en ce qu'**au moins l'espace intermédiaire, mis sous vide, entre le suremballage (4) et l'étui d'emballage (1 ; 28, 29) a une forme stérile.
